# EUROPEAN PATENT APPLICATION

(11) **EP 4 830 712 A1**
(43) Date of publication of application: **28.10.2026**
(21) Application number: 25171835.9
(22) Date of filing: 23.04.2025
(51) Int. Cl.: A61K 36/185, A61P 17/00, A61K 8/9789, A61P 17/08, A61P 17/14, A61P 43/00, A61Q 19/00

(54) **PASSIFLORA EDULIS (PASSION FRUIT) EXTRACT AS AN ENHANCER FOR STIMULATING VITAMIN D3 PRODUCTION**

(71) Applicant: SkyLab AG, 1066 Epalinges (CH)
(72) Inventor: Belous, Elena, 121309 MOSCOW (RU); Ivanova, Angelina, LONDON, NW61NE (GB); Filatov, Viktor Andreevich, 119415 MOSCOW (RU); Buzova, Valeriia Pavlovna, 248002 Kaluga Region, Kaluga City (RU)
(74) Representative: GLAWE DELFS MOLL

(57) **Abstract**

The invention relates to *Passiflora Edulis* extract for use in the stimulation and/or induction of vitamin D3 synthesis from vitamin D3 precursors in a subject and oral and/or dental care and skin care products comprising *Passiflora Edulis* extract and which are formulated for topical application in the oral cavity and skin of a subject.

## Description

### FIELD OF INVENTION

The present invention is related to the novel uses of Passiflora Edulis (Passion Fruit Extract) for induction of vitamin D3 synthesis in the soft tissues of human oral cavity and skin. The present invention relates to the field of cosmetic and medicinal products for oral care and skin care.

### BACKGROUND OF THE INVENTION

Vitamin D3 is a liposoluble vitamin, which has a lot of functions in the body including regulation of calcium metabolism and bone mineralization; in addition, vitamin D3 exhibits anti-inflammatory and immunomodulating properties (L Bishop E. et al., 2021). Gene expression of nuclear vitamin D3 receptors (VDR) has been detected in many cells of the organism, such as osteoblasts, in kidneys and small intestine, and is also characteristic for immune cells and cardiac tissue. The studies demonstrated the role of vitamin D3 in prevention of cancer, cardiovascular diseases, type 2 diabetes mellitus and even depression (Melamed ML, Michos ED, Post W, Astor B., 2008). Despite crucial functions of this vitamin in the organisms, vitamin D3 deficiency is found almost in half of the population (Holick MF., 2007).

Vitamin D3 is obtained from the diet or is synthesized from 7-dehydrocholesterol in skin exposed to UV-B in sunlight (290-315 nm) (Holick et al., 1981). Ultraviolet irradiation of 7-dehydrocholesterol facilitates photochemical cleavage of steroid B-ring between C9 and C10 atoms giving rise to previtamin D3. The efficiency of vitamin D3 production in skin depends on many factors including season, time of day, age, skin tone (melanin level), area of open body surface, use of spf-cream, latitude (the higher is latitude, the longer is distance passed by ultraviolet rays to the Earth surface, the greater is absorption by ozone layer) (Hollis B. W., 2005).

The previtamin undergoes further molecular isomerization and is converted into vitamin D3 (cholecalciferol), which immediately enters the circulation (Mazziotti G., Maffezzoni F., Giustina A., 2016). This an inactive form, which is further modified in liver and kidneys.

It should be mentioned also that vitamin D3 intake by the organism can result from consumption of herring, wild salmon, cod, mackerel and shiitake mushrooms, while vegetable food and yeast are the sources of vitamin D2 (ergocalciferol). D2 differs from D3 by the presence of double bond between C22 and C23 and methyl group at C24. These two groups exhibit similar mechanism; however, it was shown that vitamin D3 supplementation resulted in more efficient synthesis of 25-hydroxyvitamin D3 compared to D2 supplementation (Autier P., Gandini S., Mullie P. A., 2012); furthermore, D2 form demonstrates lower affinity to the transport proteins and shorter half-life (Bouillon R., Verlinden L., Verstuyf A., 2016).

In liver vitamin D3 undergoes hydroxylation at C25 by D-25-hydroxylase giving rise to 25-hydroxyvitamin D3 (calcidiol), which is a precursor of active form, and vitamin D-binding protein (DBP) shows high affinity to calcidiol (Christakos S. et al., 2010). In kidneys transport protein-25-hydroxyvitamin D3 complex is recognized by cubulin protein and transported through glomerular membrane into the cells of nephron proximal tubules by megalin protein (Verroust P. J., Christensen E. I., 2002). Then 25-hydroxyvitamin D3-megalin complex breaks down in the lysosome, and calcidiol enters the cytoplasm. The next activation step takes place here, in the cells of nephron proximal tubules, by 25-hydroxyvitamin-D-1α-hydroxylase. Hydroxylation at C1 results in formation of 1,25-dihydroxyvitamin D (1,25(OH)2D3) (the active vitamin D3 form capable to react with nuclear VDR due to the presence of 1α-OH group (Lips P., 2006). It is worth mentioning that the presence of 1α-hydroxylase is typical for many cell types including keratinocytes, hair follicle cells, osteoblasts, trophoblast and decidual membrane cells, cells of the gastrointestinal tract, lungs, testes, islets of Langerhans, central nervous system, as well as monocytes, and macrophages; but active vitamin D3 for systemic regulation is formed exactly in kidneys, while vitamin D3 formed in the other cells is involved in paracrine and autocrine regulation (Schwartz et al., 1998; Cross et al., 2001; Tangpricha et al., 2001; Holick, 2002). However, many tissues, including the skin, also contain both hydroxylases for calcitriol synthesis [T. Passeron, R. Bouillon, V. Callender, T. Cestari, T.L. Diepgen, A.C. Green, J.C. van der Pols, B.A. Bernard, F. Ly, F. Bernerd, L. Marrot, M. Nielsen, M. Verschoore, N.G. Jablonski, A.R. Young, Sunscreen photoprotection and vitamin D status, British Journal of Dermatology, Volume 181, Issue 5, 1 November 2019, Pages 916-931]. Stimulation of gene expression of 25-hydroxyvitamin-D-1α-hydroxylase, CYP27B1, occurs due to effect of parathyroid hormone (parathormone) and parathormone-related peptide under activation of receptors PTH1R (type 1 parathyroid receptor) and initiation of adenylate cyclase signaling pathway (cAMP-> PkC). Proteinkinase C (PkC) phosphorylates nuclear protein 4, MBD4 (a protein comprising type 4 methyl-CpG-binding domain), which further activates transcription acting as demethylase (Kouzmenko A. et al., 2010). Under normal blood calcium concentrations, the increase of CYP27B1 expression in nephron straight tubules is induced by calcitonin, when triggering a signaling pathway involving the transcription factor C/EBPb and the SWI/SWF chromatin remodeling complex. Higher level of 1α-hydroxylase CYP27B1 expression is observed also under hypocalcemia, hypophosphatemia; growth factor, insulin-like growth factor 1 (IGF-1) and prolactin also up-regulate the expression of this enzyme (Zhong Y, Armbrecht HJ, Christakos S., 2009). The expression of gene encoding the principal activating enzyme is down-regulated by increase in calcium, phosphate levels and by negative feedback mechanism from calcitriol (active vitamin D3 form) itself under activation of VDR receptors. The vitamin is metabolized by 24-hydroxylase, which transforms the active form (calcitriol) into inactive 1,24,25- hydroxyvitamin D.

Vitamin D3 or D2 supplements and also alimentary vitamin are packed in chilomicrons in the intestine and pass into intestinal lymphatic system and then to circulation. Both D2 and D3 form the complexes with vitamin D-binding protein (DBP), which are transported to the liver where undergo hydroxylation and transportation processes similar to endogenous vitamin D3.

Most of calcitriol circulates in the bloodstream in DBP-bound form, while free vitamin comprises only 0.4% of total calcitriol. The role of calcitriol is to affect the gene expression (about 900 of target genes) through interaction with VDR nuclear receptor (Morris, H.A.; Anderson, P.H., 2010). Calcitriol target genes encode the proteins involved in bone and mineral metabolism and also the proteins contributing to maintenance of energy metabolism, functioning of immune and cardiovascular systems, skin and muscular cells (Rosen C. J. et al., 2012). Calcitriol genomic effects develop within hours and even days because they require multistage processes taking place in different cell compartments (transcription, translation, post-translational modification, storage, transport, and secretion proteins). In addition, VDR receptors can be found in membrane caveolae (Norman A. W., 2006). The receptor activation promotes immediate non-genomic effects, which develop within seconds to minutes after receptor exposure to the ligand and include the activation of potential-dependent calcium and chlorine channels in osteoblasts, migration of endothelial cells, insulin secretion by β cells of the islets of Langerhans. The listed effects can develop due to initiation of several signaling pathways: 1) due to activation of adenylate cyclase and subsequent production of cAMP followed by activation of protein kinase A, 2) phosphoinositide signal transduction is possible, including PlC and PlD (phospholipases C and D) stimulating the acceleration of phosphoinositide metabolism, and the release of diacylglycerol (DAG) and IP3 (inositol triphosphate), which binds to receptors on endoplasmic reticulum increasing the permeability of calcium channels and leading to the release of calcium from the depot; 3) activation of Gq-coupled receptors leads to the activation of PlCβ1 (β1-type phospholipase C) and then various types of protein kinases C (α,β,γ); in addition, activation of Jun kinase and initiation of MAPK signaling pathway can occur as well (Fleet, J. C., 2004). Various signaling pathways can be initiated dependent on the cell type and prevalence of secondary messengers, but increase in cell level of calcium and initiation of phosphorylation cascades of target proteins is the common feature.

Due to flexible three-dimensional structure of calcitriol allowing to change configuration of the molecule, both genomic and non-genomic effects can occur (Chiellini, G., & DeLuca, H. F., 2011; Norman A. W., 2006). Trans-calcitriol with β-chair conformation of A-ring is recognized by nuclear VDR receptors. In the absence of ligand VDR is in cytoplasm, while upon interaction with calcitriol VDR binds to retinoid X receptor (RXR) giving rise to heterodimer, which is recognized by VDRE sensitive element (vitamin D-sensitive element) in regulatory 5'-region of target genes (Schuster I., 2011) and by expression activator proteins. Cis-calcitriol promotes the development of immediate non-genomic effects (Norman A. W., 2006).

Active form of vitamin D3 regulates the expression of genes encoding calbindin - calcium transporter protein, the proteins - subunits of calcium channels (TRPV5 in kidneys, TRPV6 in intestine), bone proteins - osteocalcin, osteopontin, collagen, alkaline phosphatase, osteoclast activator protein RANKL. Calcitriol regulates differentiation and proliferation of epiphyseal chondrocytes, contributes to cartilage matrix mineralization and bone formation, and inhibits bone resorption by osteoclasts (Xue, Y, Karaplis, A. C., Hendry, G. N., et al., 2006).

It is also important to note the impact of deficiency on skin health, as vitamin D plays a key role in protecting the skin from UV-induced damage. Vitamin D deficiency contributes to premature skin dryness, hyperpigmentation, loss of elasticity, wrinkle formation, and erythema [Chang, A. L. S., Fu, T., Amir, O. Association of facial skin aging and vitamin D levels in middle-aged white women // Cancer Causes Control. - 21, 2010. URL: https://www.ncbi.nlm.nih.gov/pmc/articles/PMC3042365/ (accessed: 19.02.2024)]. A variety of endogenous and environmental factors influence vitamin D synthesis in skin, including genetic polymorphisms, age, geographic location, sun exposure patterns, ultraviolet radiation dose, clothing [T. Passeron et al., British Journal of Dermatology, 2019, 181(5), 916-931].

The active metabolites of vitamin D3 (D3) exert a variety of antiaging and photoprotective effects on the skin. These are achieved through immunomodulation and include anti-inflammatory actions, regulation of keratinocytes proliferation, and differentiation programs to build the epidermal barrier necessary for maintaining skin homeostasis. In addition, they induce antioxidative responses, inhibit DNA damage and induce DNA repair mechanisms to attenuate premature skin aging and carcinogenesis. Therefore, active forms of vitamin D3 including its canonical (1,25(OH)2D3) and noncanonical (CYP11A1-intitated) D3 derivatives are promising agents for the prevention, attenuation, or treatment of premature skin aging [Bocheva, G.; Slominski, R.M.; Slominski, A.T. The Impact of Vitamin D on Skin Aging. Int. J. Mol. Sci. 2021, 22, 9097]. Moreover, Vitamin D deficiency is associated with the risk of psoriasis and atopic dermatitis, and several clinical/observational studies have suggested the beneficial effect of vitamin D in the therapy of these 2 inflammatory skin disorders [Umar, Meenakshi, et al. "Vitamin D and the pathophysiology of inflammatory skin diseases." Skin pharmacology and physiology 31.2 (2018): 74-86.].

In this context, however, the use of cosmetic products containing vitamin D forms is not feasible, as it is classified as an active pharmaceutical ingredient. The European Union prohibits the use of both vitamin D3 and D2 in cosmetic products (Annex II to Regulation (EC) No 1223/2009). Additionally, there are technical challenges associated with its application: vitamin D is a lipophilic molecule requiring enhanced drug delivery systems such as nanocarriers for effective skin penetration. Furthermore, vitamin D is sensitive to environmental factors including light, heat, and moisture, which may compromise its bioavailability [Janjetovic, Z.; Slominski, A.T. Promising Functions of Novel Vitamin D Derivatives as Cosmetics: A New Fountain of Youth in Skin Aging and Skin Protection. Cosmetics 2024, 11, 37].

Thus, the most promising approach involves exclusively stimulating endogenous vitamin D synthesis pathways in the skin and other tissues.

Tooth is a mineralized organ, and teeth mineralization occurs in parallel with mineralization of skeletal bones. This means that D3 hypovitaminosis is associated with disturbance of the process and leads to "rickets-like" teeth in children, i.e. to hypomineralized, immature, fragile teeth (Foster B.L., Nociti F.H., Somerman M.J., 2013; D'Ortenzio L., et al., 2018). In addition to the role in calcium metabolism and impact on bone mineralization, vitamin D3 is involved in modulation of immune response (White J.H., 2012), and interaction between vitamin D3 active form and VDR receptors up-regulates the expression of calcium-binding proteins and various proteins of extracellular matrix, such as enamelin, amelogenin, phosphoprotein dentin, sialoglycoprotein) thus contributing to dentin and enamel formation (Foster B.L., Nociti F.H., Somerman M.J., 2013; Slavkin H.C., et al., 1992).

Vitamin D plays an important role in maintenance of healthy oral cavity (Uwitonze A.M. et al., 2018). It was shown that dental caries and periodontitis are associated with vitamin D deficiency (Hujoel P.P., 2013; Schroth R.J., et al., 2013; Dietrich T., et al., 2004; Scardina G.A., Messina P., 2012). Results of the analysis of pooled 24 studies, in which 2827 children were enrolled, showed that vitamin D intake as D2 and D3 and also vitamin D production under exposure of UV-irradiation reduced the risk of tooth decay by 47% (Hujoel P.P., 2013). Another study demonstrated that children without caries are twice as likely to have optimal concentrations of vitamin D3 (≥75 nmol/L) compared to children with caries, and the most severe caries in children is associated with low vitamin D3 level (<35 nmol/L).

On the one hand, vitamin D3 cannot affect the structure of the tooth, which is already formed, on the other hand, at this stage the mechanisms of vitamin D3 influence on the immune system are activated. For instance, it was shown that in monocytes the activation of toll-like receptors (TLR) 2/1 in the presence of Mycobacterium tuberculosis and activation of VDR receptors by 1,25-dihydroxyvitamin D result in up-regulation of expression of cathelicidin antimicrobial peptide (Hewison M., 2012). Cathelicidin gene comprises vitamin D3-sensitive element (VDRE) (Gombart AF, Borregaard N, Koeffler HP., 2005; Wang TT, Nestel FP, Bourdeau V, et al., 2004). Thus, vitamin D3 indeed can contribute to the monocyte fight against pathogenic microorganisms. Furthermore, TLR activation and initiation of signaling cascades such as JAK-STAT, MAP, and transcription factor NF-kB results in up-regulation of expression of CYP27B1 encoding 1α-hydroxylase (Stoffels K, Overbergh L, Giulietti A, et al., 2006), the enzyme responsible for the last step of vitamin D3 activation. It appeared that vitamin D3 has numerous targets among the immune cells, VDR receptor is expressed on B cells, T cells, and antigen-presenting cells. Thus, vitamin D3 can modulate both innate and adaptive immune reactions.

Vitamin D3 levels are negatively correlated with the risk of periodontitis, and lower vitamin D3 concentrations are associated with more severe stages of periodontium inflammation (Anbarcioglu, E. et al., 2019, Agrawal, A.A. et al., 2019, Isola, G. et al., 2020, Antonoglou, G.N. et al., 2015). Gingival hemorrhage is also lower in persons with adequate vitamin D3 level compared to the persons with hypovitaminosis (Dietrich, T. Et al., 2004). Low vitamin D3 level is associated with higher inflammatory marker levels (IL-35, IL-17A, TGFβ) (Costantini, E. et al., 2020). Vitamin D3 exhibits an anti-inflammatory and mineralizing effect on the periodontium, and may help to reduce the population of living bacterium Porphyromonas gingivalis, which is one of the most aggressive periodontopathogens, stimulating the macrophages to active autophagy (Hu, X. et al., 2019), and also inhibits IL-6 overexpression alleviating the inflammation (Li, H., et al., 2019). In the human periodontal cell culture vitamin D3 induced mineralization comparable with vitamin C effect through increase of expression of genes encoding collagen 1 α1, alkaline phosphatase, osteocalcin, osteopontin, and other compounds involved in dental cementum formation (Hong, H. et al., 2019).

In adults vitamin D3 has indirect effect on teeth mineralization and health - high blood vitamin concentration contributes to calcium reabsorption in kidneys and small intestine and maintains the adequate calcium and phosphorus levels. Saliva is a plasma ultrafiltrate and contains the proteins synthesized in salivary glands, and for this reason saliva and blood are alike in mineral composition. Saliva calcium and phosphate ion levels influence the speed and efficacy of enamel remineralization, because in adults this process is a passive ion exchange between enamel hydroxyapatites and saliva ions.

Vitamin D3 has a direct impact on oral health and enamel integrity through stimulation of immune cells, thus contributing to the protection from pathogenic flora such as cariogenic bacteria in oral cavity. Proliferation of pathogenic flora contributes to the acid release associated with decrease in oral pH and local pH on teeth surface thus disturbing the remineralization processes, and eventually leads to decay of solid dental tissues. Therefore, timely immune response promoted by vitamin D3 indirectly prevents the enamel demineralization.

In spite of correlation between oral and blood vitamin D3 levels, the inactive form of oral vitamin D3 25(OH)D3 correlates with the blood level at 0.424, as was shown by Sari D. K. et al., 2021, while for the active form 1,25(OH2)D3 the correlation level was only 0.339. Therefore, the oral efficiency of vitamin D3 can be considerably higher in the event of local hydroxylation at C1 and formation of active vitamin form in oral cavity.

Are oral cavity cells able to synthesize locally the active vitamin D3? It was shown that mesenchymal stem cells in oral cavity express both CYP27B1, CYP27A1, and CYP24A1, i.e. the genes encoding the enzymes 1α-hydroxylase and 24-hydroxylase (Geng, S.; Zhou, S.; Glowacki, J., 2011). It was shown that the fibroblasts in oral cavity - gingiva and periodontium - express CYP27A1 and CYP27B1, this means that said fibroblasts can convert vitamin D3 to 25(OH)D3 and 25(OH)D3 to 1,25(OH)2D3, respectively (Liu, K.; Meng, H.; Hou, J., 2012a and 2012b). Therefore, the oral cavity cells are able to transform vitamin D3 locally to 25(OH)D3 and 1,25(OH)2D3. Both systemically and locally synthesized calcitriol can have a biological effect on the processes in oral cavity.

As was described earlier, vitamin D3 is an essential liposoluble vitamin, which is involved in numerous metabolic pathways. Vitamin D3 deficiency is a global problem especially in the regions remote from the equator and with few sunny days (Holick, M.F., 2017). Vitamin supplementation is a common solution of this problem, and is especially indicated in the events of vitamin D3 deficiency, metabolic disorders, to pregnant women and obese persons, for cardiovascular and autoimmune diseases, diabetes mellitus, aging, and liver diseases (Karras, S. et al., 2016; Caristia, S. et al., 2019; Perna, S. et al, 2019; Perna, S. et al, 2019; Anagnostis, P.; Paschou, S.A.; Goulis, D.G., 2018; Ojo, O. et al., 2019; Pittas, A.G. et al., 2019; Anagnostis, P. et al., 2017; Smyk, D.S. et al., 2018; Efe, C. et al., 2014; Infante, M. et al., 2019).

Many forms and routes of administration are used, for instance, capsules, gels, oral drops, buccal and sublingual sprays. It was hypothesized that vitamin bioavailability, release and absorption strongly depend on the frequency and route of administration (Dalle Carbonare, L. et al., 2018; Maurya, V.K.; Bashir, K.; Aggarwal, M. et al., 2019; Mentaverri, R. et al., 2019).

Daily intake of vitamin D3 is usually more efficacious compared to once a week or once a month administration. However, administration of high loading doses followed by the maintenance doses can be efficacious as well especially in persons with marked vitamin D3 deficiency (Chel V. et al., 2008). Oral forms showed better bioavailability and efficacy in elevation of blood active vitamin level (Chel V. et al., 2008), while intramuscular injections may provide more sustained increase in D3 levels (CHHABRA S. et al., 2022). It is also necessary to take into account that D3 is a liposoluble vitamin, which shows better bioavailability upon encapsulation into the lipid capsules (Šimoliu̅nas E. et al., 2019).

In addition, administration in combination with vitamin K is recommended. Vitamin D3 increases the expression of proteins, while activation of said proteins depends on vitamin K-mediated carboxylation, and greater demand in carboxylation is associated with depletion of vitamin K reserves. Vitamin K is important for the nervous system and plays an essential role in protection from the bone mass loss (Masterjohn C., 2007), and is involved in bone mineralization providing for γ-carboxylation of osteocalcin (Bügel S., 2003).

Despite frequently observed vitamin D3 deficiency and common advises on vitamin D supplementation, the concentration monitoring is still needed because excessive vitamin D3 may result in hypercalcemia. According to EFSA Panel on Dietetic Products, Nutrition and Allergies (NDA), 2012, the highest acceptable daily dose is 4000 International Units (IU) or 100 µg.

It is known that vitamin D3 stability depends on various factors, such as exposure to light, moisture, oxygen, temperature and pH. Aqueous vitamin solutions are not stable at pH below 5, and various metal ions (Fe2+, Cu+, 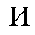 Cu2+) can catalyze vitamin D3 degradation. Rapid degradation with the loss of up to 15% of active substance per week is observed at temperatures above 40 °C (Al-Baitai A. Y. et al., 2020) and under exposure to UV irradiation (Temova Rakuša Ž. et al., 2021). Lack of control of factors that reduce the vitamin stability during the manufacturing process, violation of storage conditions, effect of the additives contributing to the active substance degradation - all these factors complicate the control over consumed vitamin D3 concentrations (Demer L.L. et al., 2018; Cesari M. et al, 2011).

Vitamin D3 is considered a medication when taken in active form, and the bans on the use of the vitamin in beauty products is based on preventive considerations since vitamin D3 exhibits hormonal effects in the organism (Cesari M. et al., 2011; Demer L. L., Hsu J. J., Tintut Y, 2018). The ban also reflects the need to control the consumed concentrations, that is hardly possible with the use of the beauty products and skincare products. However, vitamin D3 precursor 7-dehydrocholesterol is approved for addition in the beauty products (European Commission. CosIng - Cosmetics - GROWTH. Ingredient: 7-DEHYDROCHOLESTEROL. https://ec.europa.eu/growth/tools-databases/cosing/details/74131. Accessed 03.03.2025) (as stabilizer used for viscosity control). However, rapid synthesis of active vitamin D3 cannot be achieved even upon addition of the precursor because at first several steps of ring transformation in 7-dehydrocholesterol under exposure to UV-B radiation are required.

Due to the positive effect on oral health vitamin D3 is a promising ingredient of the toothpastes and other oral care products, however, implementation of this idea is precluded by the prohibition of use of vitamin D3 in the beauty products. Stimulation of local vitamin D3 production in oral cavity can be a solution. This is really possible, because, as was set forth earlier, many human cells including fibroblasts express CYP27B1, the gene of 1α-hydroxylase, which facilitates the formation of active vitamin D3 (1,25(OH)2D3 from 25(OH)D3) (Geng, S.; Zhou, S.; Glowacki, J., 2011; Liu, K.; Meng, H.; Hou, J., 2012a 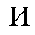 2012b). Stimulation of local vitamin D3 production can become an innovative solution for maintenance of oral health, protection from cariogenic bacteria and provision of conditions for enamel remineralization. Furthermore, with this approach there is no need to control the dosage or storage conditions because local production implies the same local and short action on the cells having VDR receptors in oral cavity.

The regulation of vitamin D3 (cholecalciferol) in cosmetics is primarily concerned with exogenous application-directly applying vitamin D3 to the skin or mucous membranes. This raises concerns about uncontrolled absorption, potential overdose, and systemic effects. In contrast, endogenous synthesis of vitamin D3 in the oral cavity (or skin) is a natural, self-regulated process. The body synthesizes vitamin D3 from 7-dehydrocholesterol under UVB exposure (sunlight), and this process is tightly controlled by feedback mechanisms. Excess vitamin D3 is not produced because the precursor molecules are limited, and the reaction stops once sufficient levels are reached (Papadopoulou S. N. A. et al., 2025; Taylor P. N., et al., 2018).

Stimulating vitamin D3 synthesis in the oral cavity would likely result in localized production, which is unlikely to lead to systemic overdose. The oral mucosa is not as efficient at producing vitamin D3 as the skin, and the amounts synthesized would be minimal compared to systemic needs (Papadopoulou S. N. A. et al., 2025). However, a randomized clinical trial demonstrated that exposure to sunlight alone has a very minimal effect on increasing blood vitamin D levels compared to dietary supplements. Therefore, even enhanced production of vitamin D3 in skin does not provide sufficient amounts of vitamin D3 in blood and certainly does not exceed normal physiological levels [Wacker, Matthias, and Michael F. Holick. "Sunlight and Vitamin D: A global perspective for health." Dermato-endocrinology 5, no. 1 (2013): 51-108. doi:10.4161/derm.24494]. Moreover, the body has built-in regulatory mechanisms (e.g., degradation of excess vitamin D3) to maintain homeostasis.

Indeed, some substances stimulating vitamin D3 synthesis or inhibiting vitamin D3 degradation are known from the literature. For instance, the studies by Cruciani S. et al., 2019, showed that the extract from pulp and seeds of myrtle (Myrtus communis L.) induced the expression of some CYP enzymes including CYP27B1, thus activating local synthesis of 1,25(OH)2D3 in fibroblasts. The synthesis of other cytochrome P450 enzymes contributed to alleviation of inflammation and protected from oxidative stress development. The other study by Trivedi M. K. et al., 2023, demonstrated that cannabidiol also stimulates the expression of CYP27B1 and VDR, i.e. vitamin D3 receptor, while decreasing the expression of CYP24A1, which is an enzyme responsible for vitamin degradation. Cao S. et al., 2018 describe the effect of oleic acid aimed at elevation of CYP27B1 mRNA level and decrease of CYP24A1 mRNA level, but exactly in the cells of proximal tubules. Curcumin and sulphoraphane (thiocyanate found in Crucifers) induce a number of epigenetic changes, which contribute to histone modification modifications and up-regulate the expression of VDR gene (vitamin D3 receptor). Furthermore, these compounds show anti-inflammatory and antioxidant activities (Abdalla M. et al., 2018; Apprato G. et al., 2020). The use of the above extracts and substances in the oral care products has not yet been described in literature.

On the one hand, vitamin D3 has immunomodulating and antioxidative effects, and in suitable conditions the local synthesis of vitamin is initiated. It is known that transcription factor NF-kB and pro-inflammatory cytokines TNF-α and IL-6 can promote the expression of CYP27B1 (Zordoky B. N. M., El-Kadi A. O. S., 2009; Noyola-Martinez N. et al., 2014). Flavonoids (for instance, vitexin, isovitexin, and quercetin), phenolic acids, and carotenoids found in the passion fruit extract have immunomodulating and antioxidative properties, i.e. they inhibit NF-kB activity and reduce the cytokine level thus suppressing the expression of enzymes responsible for the synthesis of active vitamin D3 form (Ribeiro D. et al., 2015). In addition, antioxidant activity of the extract components can hamper the oxidation reactions involved in hydroxylation for the formation of 1,25(OH2)D3.

On the other hand, decreased cytokine production and lower oxidative stress under exposure to extract help to stabilize the enzymes responsible for synthesis and metabolism of vitamin D3, including 1α-hydroxylase, which is a cytochrome P450 enzyme comprising an oxidation-sensitive heme group. Oxidative stress results in the disarrangement of heme group and the protein structure and thus in the worse enzyme capability for hydroxylation of 25(OH)D to 1,25(OH2)D3. Quercetin being one of the most prevalent flavonoids in the passion fruit extract can activate the vitamin D3 receptors, VDR (Inoue J. et al., 2010) acting in synergy with vitamin D3 (Sannappa Gowda N. G. et al., 2023). The retinoic acid precursors, β-carotenoids, are also found in the passion fruit extract. Retinoic acid interacts with RAR and RXR receptors, which are the heterodimerization counterparts of vitamin D3 receptors, and this results in potentiation of the vitamin effect and initiation of the signaling pathways (Schräder M. et al.,1993).

Of note, the vitamin C level in the passion fruit extract is very high (Fonseca A. M. A. et al., 2022), however, increase in oral vitamin C concentration will not promote the enamel remineralization, the effect will be just the opposite. Vitamin C shifts the processes of ion exchange between the enamel hydroxyapatite and saliva towards hydroxyapatite dissolution contributing to the reduction of oral pH and leading to demineralization (Meurman J. H., Murtomaa H.,1986).

Thus, the capability of the passion fruit extract to promote vitamin D3 metabolism in fibroblasts is probably mediated by indirect mechanisms including the modulation of immune response, regulation of cytokine levels, gene expression and antioxidative effects. Though vitamin D3 is not found in the passion tree extract, the other extract ingredients can stimulate enzymatic transformation of vitamin D3 precursors to the active form and modulate various signaling pathways enhancing the vitamin effect or promoting to the vitamin synthesis.

The authors hypothesized that the use of Passiflora edulis (passion fruit extract) could promote vitamin D3 synthesis in the oral cavity through indirect mechanisms, including immune response modulation, cytokine level regulation, gene expression, and antioxidant effects. Although passion fruit extract does not inherently contain vitamin D3, certain compounds within the extract may stimulate the enzymatic conversion of vitamin D3 precursors into their active form and modulate various signaling pathways that enhance vitamin D3 activity or promote its synthesis. However, this hypothesis is constrained by the lack of standardized data on the extract's composition, which makes it difficult to identify specific components potentially responsible for vitamin D3 production. Additionally, the mechanisms by which these putative components might facilitate vitamin D3 synthesis remain unexplored. Furthermore, the potential for passion fruit extract to stimulate vitamin D3 production is limited by its instability when exposed to light, which could significantly reduce the likelihood of achieving sufficient vitamin D3 release, even if such production were theoretically possible.

### Investigations

### Example 1. Determination of non-cytotoxic concentrations of Passiflora edulis (passion fruit) extract

### 1.1 Materials and methods

### 1.1.1 Investigated ingredient

**Table 1. Investigated ingredient in-vitro study**

| 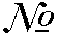 | *Ingredient* |
|---|---|
| *1* | *Passiflora edulus* (Passion fruit) extract |

### 1.1.2. Investigation

The Passiflora edulis (passion fruit) extract CAS: 91770-48-8 was obtained through a hydroglycolic extraction process using fresh whole fruits, including peel and seeds. The raw material was cleaned, dried (60-70°C), and ground into a fine powder (30-60 mesh) to enhance solvent penetration. A glycerine-water solution (30-50% glycerine by volume) served as the extraction solvent, facilitating broad-spectrum solubility of both hydrophilic and lipophilic compounds. The powdered material was subjected to reflux extraction (60-80°C, 2-4 h) at a 1:10-1:20 (w/v) solid-to-solvent ratio, followed by filtration and partial water evaporation under reduced pressure (40-60°C) to concentrate the extract. The final formulation (INCI: Aqua, Glycerin, Passiflora Edulis Fruit Extract [≥40% active constituents], Potassium Sorbate, Citric Acid) preserved a high concentration of bioactive compounds, including polyphenols (flavonoids, anthocyanins), organic acids (citric, malic), and oligosaccharides, while avoiding toxic solvents. The extract was stored in light-protected containers to maintain stability (Klinchongkon, K. et al., 2017). While the exact phytochemical profile of the commercial extract was not analytically verified in this work and was not provided by manufacture, literature reports suggest that such extracts typically contain bioactive compounds including polyphenols (e.g., gallic acid [~31.20 mg/g in peels], vitexin, and piceatannol [50-100 µg/g in seeds]), pectin (up to 37.37 g/100 g in peels), and flavonoids (e.g., chlorogenic acid, quercetin) with demonstrated antioxidant, anti-inflammatory, and anti-diabetic properties. Standardization parameters for passion fruit extracts often emphasize total phenolic content (reported as 85 mg GAE/g in peels) and pectin yield, though extraction methods (e.g., supercritical CO₂ or enzymatic treatments) significantly influence compositional variability (Weyya G. et al., 2024; Patent 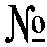 KR101976202B1; Viganó J., Martinez J., 2015).

Primary human gingival fibroblasts were seeded in 75 cm2 flasks (Corning, USA), cultured and expanded in 37°C incubator in the presence of 5% CO2. Upon reaching confluence, keratinocytes were seeded in 96 wells plates (Corning, USA) to determination of the noncytotoxic concentrations and the keratinocytes were seeded in 6 wells for further quantification of vitamin D3.

Cell viability was determined by a colorimetric method using MTT dye (3-(4,5 dimethylthiazol-2-yl)-2,5-diphenyltetrazolium; Sigma, St. Lous, MO). For the assay (Organization for Economic Co-operation and Development, 2010; Cotton, J. et al., 1997) the product was prepared in culture medium with 3% Tween 20 (Sigma) and added to the 96 well plate at a serial dilution in the range of 100.00 to 0.003 mg/mL using the dilution factor of 3.164. The human gingival fibroblasts culture was incubated for a period of 48 hours. MTT was then added to the culture at a concentration of 5mg/mL (30µL / well) and incubated for additional 4 hours. The contents of the well were removed and 100 µl of isopropanol was added for the purpose of solubilizing the formazan crystals formed by viable cells. The absorbance of each well was determined at 570 nm in Multiskan GO monochromator (Thermo Scientific, Finland). Cell viability was expressed as a percentage and calculated according to the equation (Syed, D. N. et al., 2012; Lee, J. H. et al., 2002): $% de viability = \frac{{OD}_{PA}}{{OD}_{NC}} \times 100 ,$ where *OD_{PA}* - product assessed optical density, *OD_{NC}* - negative control optical density.

Gingival fibroblasts were incubated with three non-cytotoxic concentrations of Passiflora edulis (Passion Fruit) extract (10.01 mg/mL, 3.17 mg/mL, and 1.00 mg/mL), as determined by the MTT assay. The cells were maintained in contact with the extract for a period of 72 hours to evaluate the effects on vitamin D3 determination. The MTT assay confirmed that the concentrations used were non-cytotoxic, ensuring cell viability and functionality throughout the experiment.

### 1.2. Results

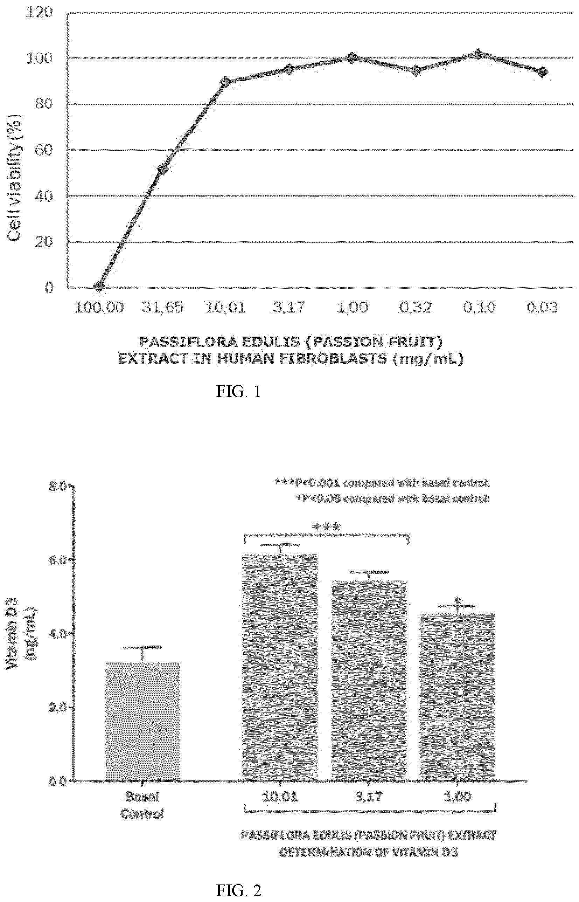

The results are illustrated in Figure 1, which shows the evaluation of the non-cytotoxic concentrations of the products assessed *Passiflora edulis* (Passion fruit) extract in human fibroblasts after 48 hours of incubation by the MTT method. As illustrated in Figure 1, *Passiflora edulis* (Passion fruit) extract demonstrated non-cytotoxic properties starting from the highest tested concentration of 10.01 mg/mL. This concentration, along with the subsequent dilutions (3.17 mg/mL and 1.00 mg/mL), was selected to assess the impact of the extract on vitamin D3 levels in human gingival fibroblasts. The evaluation of non-cytotoxic concentrations was further validated after 48 hours of incubation using the MTT method, which confirmed that the extract did not adversely affect cell viability at the tested concentrations.

The results indicate that *Passiflora edulis* (Passion fruit) extract, at the specified concentrations, is suitable for further investigation regarding its potential role in modulating vitamin D3 levels in gingival fibroblasts. The non-cytotoxic nature of the extract at these concentrations provides a safe range for exploring its biological effects, particularly in the context of vitamin D3 metabolism and its implications for oral health.

The findings suggest that *Passiflora edulis* (Passion fruit) extract, at concentrations of 10.01 mg/mL, 3.17 mg/mL, and 1.00 mg/mL, is non-cytotoxic to gingival fibroblasts and can be safely used for further studies on vitamin D3 determination. Thus, the use of the extract is limited to non-cytotoxic concentrations, namely 10,01 mg/mL or below.

**Example 2.** In-vitro Evaluation of The Effect *of Passiflora edulis* (Passion fruit) extract on Vitamin D3 Synthesis In Human gingival fibroblasts Culture.

### 2.1. Materials and methods

### 2.1.1. Investigated ingredient

**Table 2. Investigated ingredient in-vitro study**

| 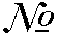 | *Ingredient* |
|---|---|
| *1* | *Passiflora edulus* (Passion fruit) extract |

### 2.1.2. Investigation

Study objective was to evaluate the preclinical effect of the evaluated products on vitamin D3 production in human gingival fibroblasts culture. After the evaluation *of Passiflora edulis* (Passion fruit) extract non-cytotoxic concentrations, the supernatant was collected for quantification of the vitamin D3.

Concentrations of Vitamin D3 were measured in the supernatant by immunoassay, accordingly to the protocol on the datasheet provided by the supplier, using a commercially available kit (Elabscience, USA). The 450 nm absorbance reading was performed in Multiskan GO monochromator (Thermo Fisher Scientific, Finland).

To evaluate the data obtained, the ANOVA test was used, which also allowed measuring the variation in results, comparing data between groups. The Bonferroni post-test was then applied, which reinforced and made the result presented in the ANOVA test even more precise. A significance level of 5% was used in both assessments.

### 2.2.Results

The results are illustrated in Figure 2, which shows the effects of the product assessed *Passiflora edulis* (Passion fruit) extract in 3 non-cytotoxic concentrations on the quantification of vitamin D3 in human gingival fibroblasts if visualization form. Data represent the mean ± standard deviation of 3 replicates (ANOVA, Bonferroni). The results demonstrated a significant impact of *Passiflora edulis* extract on vitamin D3 production in human gingival fibroblasts, as illustrated in Figure 2.

The illustration of Figure 2 is based on the original data as provided in below Table 1 showing that the *Passiflora edulis* extract significantly enhanced vitamin D3 quantification in a concentration-dependent manner. At concentrations of 10.01 mg/mL, 3.17 mg/mL, and 1.00 mg/mL, the extract increased vitamin D3 levels by 89.86% (p<0.001), 68.10% (p<0.001), and 40.65% (p<0.05), respectively, compared to the basal control. These findings indicate a robust and dose-dependent stimulatory effect of the extract on vitamin D3 production in human gingival fibroblasts.

**Table 1. Effects of the product assessed Passiflora edulis (Passion fruit) extract determination of vitamin D3 in 3 non-cytotoxic concentrations on the quantification of vitamin D3 in human gingival fibroblasts in table form. Data represent the mean ± standard deviation of 3 replicates (ANOVA, Bonferroni).**

| | | ***PASSIFLORA EDULIS* (PASSION FRUIT) EXTRACT DETERMINATION OF VITAMIN D3** | | |
|---|---|---|---|---|
| **Vitamin D3** | **BASAL CONTROL** | **10.01** | **3.17** | **1.00** |
| | 3.921 | 5.779 | 5.117 | 4.281 |
| | 3.207 | 6.162 | 5.456 | 4.565 |
| | 2.623 | 6.571 | 5.818 | 4.868 |
| **Average (ng/mL)** | **3.250** | **6.171** | **5.464** | **4.571** |
| Standard Deviation | 0.650 | 0.396 | 0.351 | 0.293 |

The results of this study suggest that *Passiflora edulis* (Passion Fruit) extract has a beneficial effect on enhancing vitamin D3 production in human gingival fibroblasts. The observed increases in vitamin D3 levels at all tested concentrations highlight the potential of this extract as a therapeutic agent for improving vitamin D3 metabolism, particularly in the context of oral health and gingival tissue maintenance.

**Example 3.** Evaluation of the effect of Passiflora edulis (Passion fruit) on vitamin D3 synthesis in human skin keratinocytes culture in vitro.

Human skin keratinocytes cultures were incubated with 3 non-cytotoxic concentrations of and Passiflora edulis (Passion fruit) extract for further vitamin D3 quantification through immunoenzymatic assay (ELISA).

Cell viability was determined by a colorimetric method using MTT dye (3-(4,5 dimethylthiazol-2-yl)-2,5-diphenyltetrazolium; Sigma, St. Lous, MO). For the assay the product was prepared in culture medium with 3% Tween 20 (Sigma) and added to the 96 well plate at a serial dilution in the range of 100.00 to 0.003 mg/mL using the dilution factor of 3.164. The human skin keratinocytes culture was incubated for a period of 48 hours. MTT was then added to the culture at a concentration of 5mg/mL (30µL / well) and incubated for additional 4 hours. The contents of the well were removed and 100 µl of isopropanol was added for the purpose of solubilizing the formazan crystals formed by viable cells. The absorbance of each well was determined at 570 nm in Multiskan GO monochromator (Thermo Scientific, Finland).

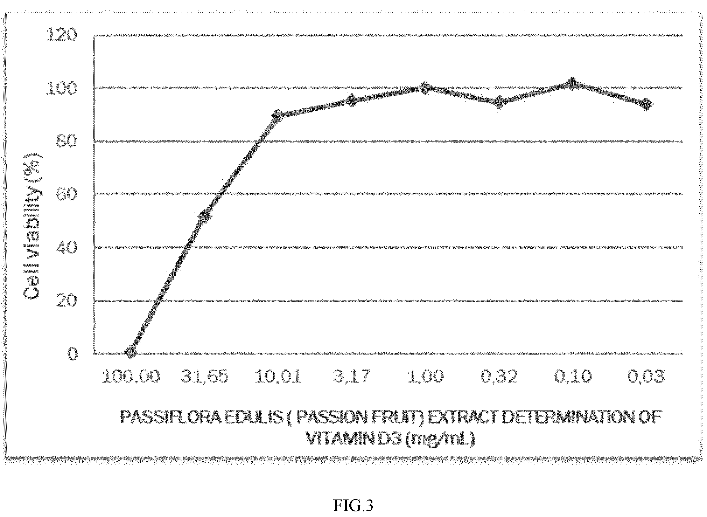

Passiflora edulis (Passion fruit) extract showed non-cytotoxic concentrations from the dilution of 10.01 mg/mL (Figure 3).

Primary human keratinocytes were seeded in 75 cm2 flasks (Corning, USA), cultured and expanded in 37°C incubator in the presence of 5% CO2. Upon reaching confluence, keratinocytes were seeded in 6 wells for further quantification of vitamin D3.

### Results

### 3.1. Quantification of vitamin D3 in human skin keratinocytes after treatment.

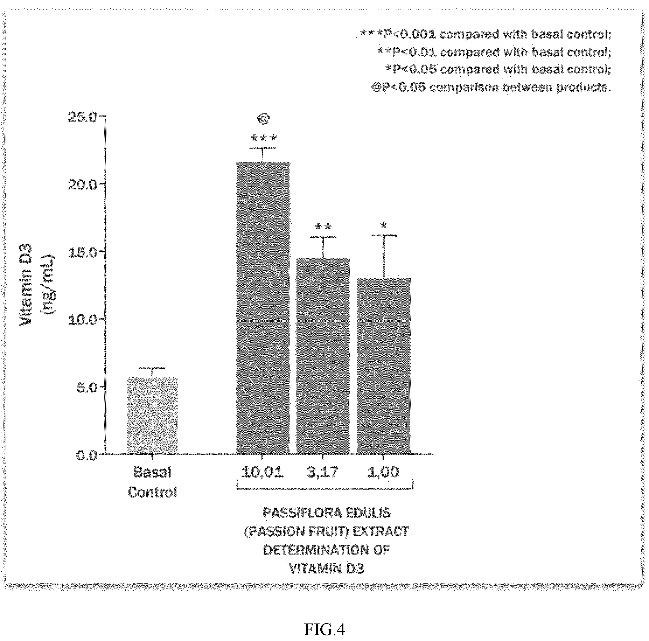

Passiflora edulis (Passion fruit) extract promoted an increase of 274.04%(p<0.001), 151.22%(p<0.01) and 125.68%(p<0.05) on vitamin D3 quantification when compared to the basal control at concentrations of 10.01, 3.17 and 1.00 mg/mL, respectively (Figure 4).

### 3.2. Quantification of vitamin D3 in human skin keratinocytes after treatment and UVB radiation exposure

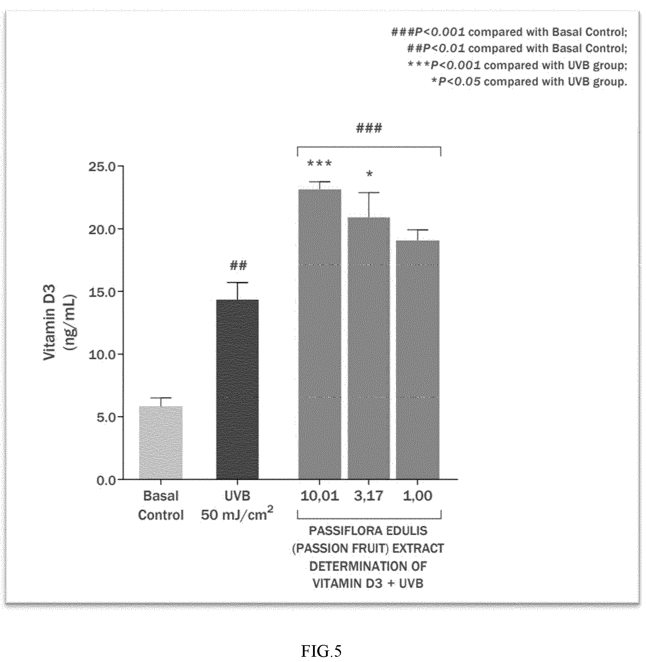

Passiflora edulis (Passion fruit) extract demonstrated significant impacts, showing increases of 297.95% (p<0.001), 259.94% (p<0.001), and 227.83% (p<0.001) in vitamin D3 quantification at concentrations of 10.01, 3.17, and 1.00 mg/mL, respectively, compared to the basal control. Regarding the quantification compared to the UVB control, the treatment showed a significant increases of 61.41% (p<0.001) and 45.82% (p<0.05), at the concentration of 10.01 and 3.17 mg/mL (Figure 5).

The results demonstrate that the Passiflora edulis (Passion fruit) extract has a beneficial effect improving the Vitamin D3 production, with or without UVB radiation.

The results obtained by the authors demonstrate clearly that the Passion fruit extract not only acts as a natural booster for the endogenous synthesis of vitamin D3 in the skin upon UV-B exposure but also appears to enable vitamin D3 synthesis even in the absence of sunlight, which is a novel scientific finding. The authors hypothesise that the incorporation of the Passion fruit extract into cosmetic formulations could enhance the versatility of the product, given its efficacy both in the presence and absence of solar radiation. The extract's dual functionality, enhancing both vitamin D synthesis and the skin's SPF protection, underscores its potential as a multifaceted skincare agent.

**Example 4.** Evaluation of the Passion fruit extract's efficacy in stimulating vitamin D production in skin ex vivo under conditions reflecting realistic cosmetic product use.

Skin fragments of approximately 1.5 cm² were carefully weighed and incubated in a culture medium to maintain viability. Each fragment was then treated with 1% of Passion fruit extract, applied at a concentration of 25 mg/cm².

Immediately after product application, the treated fragments were exposed to UVB radiation at a dose of 350 mJ/cm² using the Handisol device (National Biological Corporation, Twinsburg, OH, USA). This procedure was conducted to evaluate the effects of Passion fruit extract by comparing irradiated and non-irradiated fragments subjected to the same treatment. The treatment and UVB exposure cycle were performed once daily for three consecutive days to approximate experimental conditions to realistic sunscreen usage parameters.

Concentrations of Vitamin D3 were measured in the supernatant by immunoassay, accordingly, to the protocol on the datasheet provided by the supplier, using a commercially available kit (Elabscience, USA). The 450 nm absorbance was reading performed in Multiskan GO monochromator (Thermo Fisher Scientific, Finland).

### Results

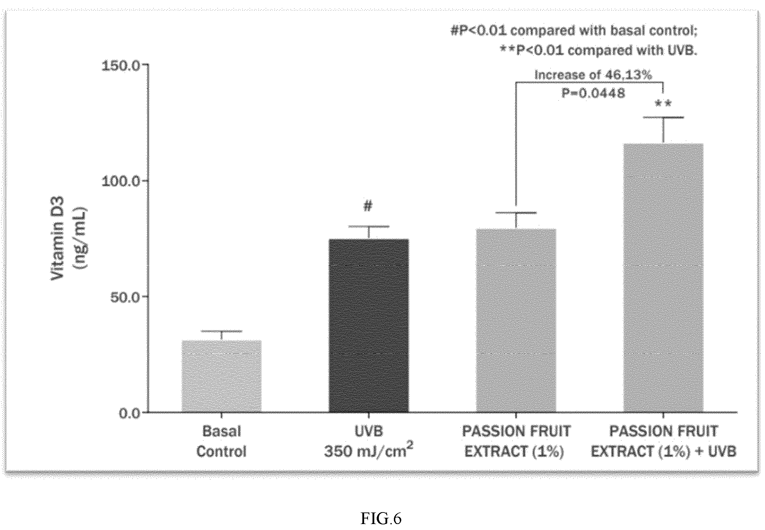

As anticipated, exposure to UVB radiation resulted in a significant 139.09% increase (p<0.01) in vitamin D3 quantification compared to the basal control (Figure 6).

The combination of passion fruit extract and UVB radiation exhibited substantial impacts, evidenced by an increase of 54.82% (p<0.01) in vitamin D3 quantification compared to the UVB control and 270.15% (p<0.01) in vitamin D3 quantification compared to the basal control (Figure 6). The Passion fruit extract, when applied without UVB radiation, exhibited a comparable effect on vitamin D synthesis to the UVB control, suggesting its efficacy in maintaining vitamin D levels in regions or seasons when sunlight is limited.

In a complementary manner, a comparison of both Passion fruit extract treatments reveals that the one associated with UVB radiation resulted in a Vitamin D3 quantification 46.13% higher than the one without UVB (Figure 6). This finding serves to underscore the synergistic effect of the Passion fruit extract in conjunction with the natural process of vitamin D synthesis induced by UV radiation from sunlight.

### References

Abdalla M. et al. Vitamin D receptor gene methylation in hepatocellular carcinoma //Gene. - 2018. - T. 653. - C. 65-71.
Agrawal, A.A.; Kolte, A.P.; Kolte, R.A.; Chari, S.; Gupta, M.; Pakhmode, R. Evaluation and comparison of serum vitamin D and calcium levels in periodontally healthy, chronic gingivitis and chronic periodontitis in patients with and without diabetes mellitus-a cross-sectional study. Acta Odontol. Scand. 2019, 77, 592-599.
Al-Baitai A. Y. et al. The effect of temperature and storage time on the concentration of vitamin D in human serum //Systematic Reviews in Pharmacy. - 2020. - T. 11. - 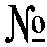 . 4. - C. 695-700.
Alshouibi E. N. et al. Vitamin D and periodontal health in older men //Journal of dental research. - 2013. - T. 92. - 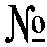 . 8. - C. 689-693.
Anagnostis, P.; Paschou, S.A.; Goulis, D.G. Calcium and Vitamin D Supplements and Fractures in Community-Dwelling Adults. JAMA 2018, 319, 2041.
Anagnostis, P.; Paschou, S.A.; Goulis, D.G. Vitamin D Supplementation and Cardiovascular Disease Risk. JAMA Cardiol. 2017, 2, 1281.
Anbarcioglu, E.; Kirtiloglu, T.; Öztürk, A.; Kolbakir, F.; Acikgöz, G.; Colak, R. Vitamin D deficiency in patients with aggressive periodontitis. Oral Dis. 2019, 25, 242-249
Antonoglou, G.N.; Knuuttila, M.; Niemelä, O.; Raunio, T.; Karttunen, R.; Vainio, O.; Hedberg, P.; Ylöstalo, P.; Tervonen, T. Low serum level of 1,25(OH)2D is associated with chronic periodontitis. J. Periodontal Res. 2015, 50, 274-280.
Apprato G. et al. Natural epigenetic modulators of vitamin D receptor //Applied sciences. - 2020. - T. 10. - 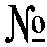 . 12. - C. 4096.
Autier P., Gandini S., Mullie P. A systematic review: influence of vitamin D supplementation on serum 25-hydroxyvitamin D concentration //The Journal of Clinical Endocrinology & Metabolism. - 2012. - T. 97. - 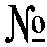 . 8. - C. 2606-2613.
Bassatne, A.; Chakhtoura, M.; Saad, R.; Fuleihan, G.E.-H. Vitamin D supplementation in obesity and during weight loss: A review of randomized controlled trials. Metabolism 2019, 92, 193-205.
Bouillon R., Verlinden L., Verstuyf A. Is vitamin D2 really bioequivalent to vitamin D3?. - 2016.
Bügel S. Vitamin K and bone health //Proceedings of the Nutrition Society. - 2003. - T. 62. - 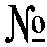 . 4. - C. 839-843.
Cao S. et al. Oleanolic acid exerts osteoprotective effects and modulates vitamin D metabolism //Nutrients. - 2018. - T. 10. - 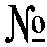 . 2. - C. 247.
Caristia, S.; Filigheddu, N.; Barone-Adesi, F.; Sarro, A.; Testa, T.; Magnani, C.; Aimaretti, G.; Faggiano, F.; Marzullo, P. Vitamin D as a Biomarker of Ill Health among the Over-50s: A Systematic Review of Cohort Studies. Nutrients 2019, 11, 2384.
Cesari M. et al. Vitamin D hormone: a multitude of actions potentially influencing the physical function decline in older persons //Geriatrics & gerontology international. - 2011. - T. 11. - 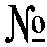 . 2. - C. 133-142.
Chel V. et al. Efficacy of different doses and time intervals of oral vitamin D supplementation with or without calcium in elderly nursing home residents //Osteoporosis International. - 2008. - T. 19. - C. 663-671.
CHHABRA S. et al. Comparing the Effectiveness of Oral versus Intramuscular Vitamin D Supplementation in Adults with Fracture around Hip and Vitamin D Deficiency //Journal of Clinical & Diagnostic Research. - 2022. - T. 16. - 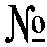 . 6.
Chiellini, G., & DeLuca, H. F. (2011). The importance of stereochemistry on the actions of vitamin D. Curr Top Med Chem, 11, 840-859
Christakos S. et al. Vitamin D: metabolism //Endocrinology and Metabolism Clinics. - 2010. - T. 39. - 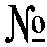 . 2. - C. 243-253.
Costantini, E.; Sinjari, B.; Piscopo, F.; Porreca, A.; Reale, M.; Caputi, S.; Murmura, G. Evaluation of salivary cytokines and Vitamin D levels in periodontopathic patients. Int. J. Mol. Sci. 2020, 21, 2669.
Cotton, J., & Spandau, D. F. (1997). Ultraviolet B-Radiation Dose Influences the Induction of Apoptosis and p53 in Human gingival fibroblasts. Radiation Research, 147(2), 148. doi:10.2307/3579415
Cruciani S. et al. Myrtus polyphenols, from antioxidants to anti-inflammatory molecules: exploring a network involving cytochromes P450 and vitamin D //Molecules. - 2019. - T. 24. - 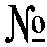 . 8. - C. 1515.
D'Ortenzio L., Kahlon B., Peacock T., Salahuddin H., Brickley M. The rachitic tooth: Refining the use of interglobular dentine in diagnosing vitamin D deficiency. Int. J. Paleopathol. 2018;22:101-108. doi: 10.1016/j.ijpp.2018.07.001
Dalle Carbonare, L.; Valenti, M.; del Forno, F.; Piacentini, G.; Pietrobelli, A. Vitamin D Daily versus Monthly Administration: Bone Turnover and Adipose Tissue Influences. Nutrients 2018, 10, 1934.
Demer L. L., Hsu J. J., Tintut Y. Steroid hormone vitamin D: implications for cardiovascular disease //Circulation research. - 2018. - T. 122. - 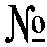 . 11. - C. 1576-1585.
Dietrich T. et al. Association between serum concentrations of 25-hydroxyvitamin D and gingival inflammation //The American journal of clinical nutrition. - 2005. - T. 82. - 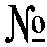 . 3. - C. 575-580.Temova Rakuša Ž. et al. Comprehensive stability study of vitamin D3 in aqueous solutions and liquid commercial products //Pharmaceutics. - 2021. - T. 13. - 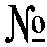 . 5. - C. 617.
Dietrich T., Joshipura K.J., Dawson-hughes B., Bischoff-ferrari H.A. Association between serum concentrations of 25-hydroxyvitamin D 3 and periodontal disease in the US population 1-3. Am. J. Clin. Nutr. 2004;80:108-113.
Efe, C.; Kav, T.; Aydin, C.; Cengiz, M.; Imga, N.N.; Purnak, T.; Smyk, D.S.; Torgutalp, M.; Turhan, T.; Ozenirler, S.; et al. Low Serum Vitamin D Levels Are Associated with Severe Histological Features and Poor Response to Therapy in Patients with Autoimmune Hepatitis. Dig. Dis. Sci. 2014, 59, 3035-3042.
EFSA Panel on Dietetic Products, Nutrition and Allergies (NDA). Scientific opinion on the tolerable upper intake level of vitamin D //EF SA Journal. - 2012. - T. 10. - 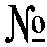 . 7. - C. 2813.
Fleet, J. C. (2004). Rapid, membrane-initiated actions of 1,25 dihydroxyvitamin D: what are they and what do they mean? J Nutrition, 134, 3215-3218.
Fonseca A. M. A. et al. Purple passion fruit (Passiflora edulis f. edulis): A comprehensive review on the nutritional value, phytochemical profile and associated health effects //Food Research International. - 2022. - T. 160. - C. 111665.
Foster B.L., Nociti F.H., Somerman M.J. The rachitic tooth. Endocr. Rev. 2014;35:1-34. doi: 10.1210/er.2013-1009.
Geng, S.; Zhou, S.; Glowacki, J. Effects of 25-hydroxyvitamin D(3) on proliferation and osteoblast differentiation of human marrow stromal cells require CYP27B1/1alphahydroxylase. J. Bone Miner. Res. 2011, 26, 1145-1153.
Gombart AF, Borregaard N, Koeffler HP. Human cathelicidin antimicrobial peptide (CAMP) gene is a direct target of the vitamin D receptor and is strongly up-regulated in myeloid cells by 1,25-dihydroxyvitamin D3. FASEB J 2005;19:1067.
He X. et al. Passiflora edulis: an insight into current researches on phytochemistry and pharmacology //Frontiers in pharmacology. - 2020. - T. 11. - C. 617.
Hewison M. Vitamin D and the immune system: new perspectives on an old theme //Rheumatic Disease Clinics. - 2012. - T. 38. - 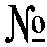 . 1. - C. 125-139.
Holick MF, MacLaughlin JA, Doppelt SH. 1981. Regulation of cutaneous previtamin D3 photosynthesis in man: Skin pigment is not an essential regulator. Science 211:590- 593
Holick MF. 1994. Vitamin D: New horizons for the 21st century. Am J Clin Nutr 60:619-630
Holick MF. Vitamin D deficiency. N Engl J Med 2007;357:266-81.
Holick, M. F., & Garabedian, M. (2006). Vitamin D photobiology, metabolism, mechanism of action, and clinical application. In M. J. Favus (Ed.), Primer on the metabolic bone diseases and disorders of mineral metabolism (pp. 106-114) (6th ed.). Washington, DC: American Society for Bone and Mineral Research
Holick, M.F. The vitamin D deficiency pandemic: Approaches for diagnosis, treatment and prevention. Rev. Endocr. Metab. Disord. 2017, 18, 153-165.
Hollis B. W. Circulating 25-hydroxyvitamin D levels indicative of vitamin D sufficiency: implications for establishing a new effective dietary intake recommendation for vitamin D //The Journal of nutrition. - 2005. - T. 135. - 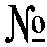 . 2. - C. 317-322.
Hong, H.; Hong, A.; Wang, C.; Huang, E.; Chiang, C. Calcitriol exerts a mineralization-inductive effect comparable to that of vitamin C in cultured human periodontium cells. Am. J. Transl. Res. 2019, 11, 2304-2316.
Hu, X.; Niu, L.; Ma, C.; Huang, Y; Yang, X.; Shi, Y; Pan, C.; Liu, J.; Wang, H.; Li, Q.; et al. Calcitriol decreases live Porphyromonas gingivalis internalized into epithelial cells and monocytes by promoting autophagy. J. Periodontol. 2019.
Hujoel P.P. Vitamin D and dental caries in controlled clinical trials: Systematic review and meta-analysis. Nutr. Rev. 2013;71:88-97. doi: 10.1111/j.1753-4887.2012.00544.x.
Infante, M.; Ricordi, C.; Sanchez, J.; Clare-Salzler, M.J.; Padilla, N.; Fuenmayor, V; Chavez, C.; Alvarez, A.; Baidal, D.; Alejandro, R.; et al. Influence of Vitamin D on Islet Autoimmunity and Beta-Cell Function in Type 1 Diabetes. Nutrients 2019, 11, 2185.
Inoue J. et al. Quercetin enhances VDR activity, leading to stimulation of its target gene expression in Caco-2 cells //Journal of nutritional science and vitaminology. - 2010. - T. 56. - 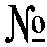 . 5. - C. 326-330
Isola, G.; Alibrandi, A.; Rapisarda, E.; Matarese, G.; Williams, R.C.; Leonardi, R. Association of vitamin D in patients with periodontitis: A cross-sectional study. J. Periodontal Res. 2020, 1-11.
Karras, S.; Paschou, S.A.; Kandaraki, E.; Anagnostis, P.; Annweiler, C.; Tarlatzis, B.C.; Hollis, B.W.; Grant, W.B.; Goulis, D.G. Hypovitaminosis D in pregnancy in the Mediterranean region: A systematic review. Eur. J. Clin. Nutr. 2016, 70, 979-986.
Klinchongkon K. et al. Extraction of oligosaccharides from passion fruit peel by subcritical water treatment //Journal of Food Process Engineering. - 2017. - T. 40. - 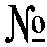 . 1. - C. e12269.
Kouzmenko A. et al. Hormonal gene regulation through DNA methylation and demethylation //Epigenomics. - 2010. - T. 2. - 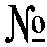 . 6. - C. 765-774.
Kulie T. et al. Vitamin D: an evidence-based review //The Journal of the American Board of Family Medicine. - 2009. - T. 22. - 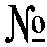 . 6. - C. 698-706.
L Bishop E. et al. Vitamin D and immune regulation: antibacterial, antiviral, anti-inflammatory //Journal of Bone and Mineral Research plus. - 2021. - T. 5. - 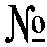 . 1. - C. e10405.
Lee, J. H., An, H. T., Chung, J. H., Kim, K. H., Eun, H. C., & Cho, K. H. (2002). Acute effects of UVB radiation on the proliferation and differentiation of keratinocytes. Photodermatology, Photoimmunology and Photomedicine, 18(5), 253-261. doi:10.1034/j.1600- 0781.2002.02755
Li, H.; Zhong, X.; Li, W.; Wang, Q. Effects of 1,25-dihydroxyvitamin on experimental periodontitis and ahr/nf-κb/nlrp3 inflammasome pathway in a mouse model. J. Appl. Oral Sci. 2019, 27, 1-10.
Lips P. Vitamin D physiology //Progress in biophysics and molecular biology. - 2006. - T. 92. - 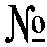 . 1. - C. 4-8.
Liu, K.; Meng, H.; Hou, J. Activity of 25-hydroxylase in human gingival fibroblasts and periodontal ligament cells. PLoS ONE 2012a, 7, e52053.
Liu, K.; Meng, H.; Hou, J. Characterization of the autocrine/paracrine function of vitamin D in human gingival fibroblasts and periodontal ligament cells. PLoS ONE 2012b, 7, e39878.
Masterjohn C. Vitamin D toxicity redefined: vitamin K and the molecular mechanism //Medical hypotheses. - 2007. - T. 68. - 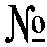 . 5. - C. 1026-1034.
Maurya, V.K.; Bashir, K.; Aggarwal, M. Vitamin D microencapsulation and fortification: Trends and technologies. J. Steroid Biochem. Mol. Biol. 2019, 105489. [CrossRef]
Mazziotti G., Maffezzoni F., Giustina A. Vitamin D-binding protein: one more piece in the puzzle of acromegalic osteopathy? //Endocrine. - 2016. - T. 52. - C. 183-186.
Melamed ML, Michos ED, Post W, Astor B. 25-hydroxyvitamin D levels and the risk of mortality in the general population. Arch Intern Med 2008;168:1629-37.).
Mentaverri, R.; Souberbielle, J.-C.; Brami, G.; Daniel, C.; Fardellone, P. Pharmacokinetics of a New Pharmaceutical Form of Vitamin D3 100,000 IU in Soft Capsule. Nutrients 2019, 11, 703.
Meurman J. H., Murtomaa H. Effect of effervescent vitamin C preparations on bovine teeth and on some clinical and salivary parameters in man //European Journal of Oral Sciences. - 1986. - T. 94. - 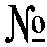 . 6. - C. 491-499
Morris, H.A.; Anderson, P.H. Autocrine and paracrine actions of vitamin d. Clin. Biochem. Rev. 2010, 31, 129-138.
Norman A. W. Vitamin D receptor: new assignments for an already busy receptor //Endocrinology. - 2006. - T. 147. - 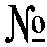 . 12. - C. 5542-5548.
Noyola-Martinez N. et al. Regulation of CYP27B1 and CYP24A1 gene expression by recombinant pro-inflammatory cytokines in cultured human trophoblasts //The Journal of steroid biochemistry and molecular biology. - 2014. - T. 144. - C. 106-109
OECD (Organization for Economic Co-operation and Development) 129 Guidance Document on Using Cytotoxicity Tests to Estimate Starting Doses for Acute Oral Systemic Toxicity Tests. OECD Environment, Health and Safety Publications Series on Testing and Assessment, Paris, 2010.
Ojo, O.; Weldon, S.M.; Thompson, T.; Vargo, E.J. The Effect of Vitamin D Supplementation on Glycaemic Control in Women with Gestational Diabetes Mellitus: A Systematic Review and Meta-Analysis of Randomised Controlled Trials. Int. J. Environ. Res. Public Health 2019, 16, 1716.
Papadopoulou S. N. A. et al. A Comprehensive Review on the Beneficial Roles of Vitamin D in Skin Health as a Bio-Functional Ingredient in Nutricosmetic, Cosmeceutical, and Cosmetic Applications //Applied Sciences. - 2025. - T. 15. - 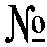 . 2. - C. 796.
Patent 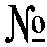 KR101976202B1. Pharmaceutical composition comprising the extracts of passion fruit rind as an effective component for prevention or treatment of thrombosis and health functional food comprising the same, 2017. URL: https://patents.google.com/patent/KR101976202B1/en
Perna, S. Is Vitamin D Supplementation Useful for Weight Loss Programs? A Systematic Review and Meta-Analysis of Randomized Controlled Trials. Medicina (B. Aires) 2019, 55, 368.
Pittas, A.G.; Dawson-Hughes, B.; Sheehan, P.; Ware, J.H.; Knowler, W.C.; Aroda, VR.; Brodsky, I.; Ceglia, L.; Chadha, C.; Chatterjee, R.; et al. Vitamin D Supplementation and Prevention of Type 2 Diabetes. N. Engl. J. Med. 2019, 381, 520-530.
Ribeiro D. et al. Proinflammatory pathways: the modulation by flavonoids //Medicinal research reviews. - 2015. - T. 35. - 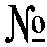 . 5. - C. 877-936
Rosen C. J. et al. The nonskeletal effects of vitamin D: an Endocrine Society scientific statement //Endocrine reviews. - 2012. - T. 33. - 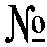 . 3. - C. 456-492.
Sannappa Gowda N. G. et al. Quercetin activates vitamin D receptor and ameliorates breast cancer induced hepatic inflammation and fibrosis //Frontiers in Nutrition. - 2023. - T. 10. - C. 1158633
Sari D. K. et al. The moderate correlation between 25 (OH) D serum and saliva in healthy people with low vitamin D intake //International journal of general medicine. - 2021. - C. 841-850.
Scardina G.A., Messina P. Good oral health and diet. J. Biomed. Biotechnol. 2012;2012:1-8. doi: 10.1155/2012/720692.
Schräder M. et al. Interaction between retinoic acid and vitamin D signaling pathways //Journal of Biological Chemistry. - 1993. - T. 268. - 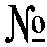 . 24. - C. 17830-17836
Schuster I. Cytochromes P450 are essential players in the vitamin D signaling system //Biochimica et Biophysica Acta (BBA)-Proteins and Proteomics. - 2011. - T. 1814. - 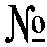 . 1. - C. 186-199.
Šimoliu̅nas E. et al. Bioavailability of different vitamin D oral supplements in laboratory animal model //Medicina. - 2019. - T. 55. - 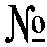 . 6. - C. 265.
Slavkin H.C., Hu C.C., Sakakura Y, Diekwisch T., Chai Y, Mayo M., Bringas P., Simmer J., Mak G., Sasano Y., et al. Gene expression, signal transduction and tissue-specific biomineralization during mammalian tooth development. Crit. Rev. Eukaryot. Gene Expr. 1992;2:315-329.
Smyk, D.S.; Mavropoulos, A.; Mieli-Vergani, G.; Vergani, D.; Lenzi, M.; Bogdanos, D.P. The Role of Invariant NKT in Autoimmune Liver Disease: Can Vitamin D Act as an Immunomodulator? Can. J. Gastroenterol. Hepatol. 2018, 2018, 8197937
Stoffels K, Overbergh L, Giulietti A, et al. Immune regulation of 25-hydroxyvitamin-d(3)-1alpha-hydroxylase in human monocytes. J Bone Miner Res 2006; 21:37
Syed, D. N., Afaq, F., & Mukhtar, H. (2012). Differential Activation of Signaling Pathways by UVA and UVB Radiation in Normal Human Epidermal Keratinocytes†. Photochemistry and Photobiology, 88(5), 1184-1190. doi:10.1111/j.1751-1097.2012.01115.
Taylor PN, Davies JS. A review of the growing risk of vitamin D toxicity from inappropriate practice. Br J Clin Pharmacol. 2018 Jun;84(6):1121-1127. doi: 10.1111/bcp.13573. Epub 2018 Apr 16. PMID: 29498758; PMCID: PMC5980613.
Trivedi M. K. et al. Effects of cannabidiol interactions with CYP2R1, CYP27B1, CYP24A1, and vitamin D3 receptors on spatial memory, pain, inflammation, and aging in vitamin D3 deficiency diet-induced rats //Cannabis and cannabinoid research. - 2023. - T. 8. - 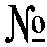 . 6. - C. 1019-1029.
Uwitonze A.M., Murererehe J., Ineza M.C., Harelimana E.I., Nsabimana U., Uwambaye P., Gatarayiha A., Haq A., Razzaque M.S. Effects of vitamin D status on oral health. J. Steroid Biochem. Mol. Biol. 2018;175:190-194. doi: 10.1016/j.jsbmb.2017.01.020.
Verroust P. J., Christensen E. I. Megalin and cubilin-the story of two multipurpose receptors unfolds //Nephrology Dialysis Transplantation. - 2002. - T. 17. - 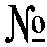 . 11. - C. 1867-1871.
Viganó J., Martinez J. Trends for the application of passion fruit industrial by-products: A review on the chemical composition and extraction techniques of phytochemicals //Food and public health. - 2015
Wang T. T. et al. Cutting edge: 1, 25-dihydroxyvitamin D3 is a direct inducer of antimicrobial peptide gene expression //The Journal of Immunology. - 2004. - T. 173. - 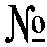 . 5. - C. 2909-2912.
Weyya G., Belay A., Tadesse E. Passion fruit (Passiflora edulis Sims) by-products as a source of bioactive compounds for non-communicable disease prevention: extraction methods and mechanisms of action: a systematic review //Frontiers in Nutrition. - 2024. - T. 11. - C. 1340511.
White J.H. Vitamin D metabolism and signaling in the immune system. Rev. Endocr. Metab. Disord. 2012;13:21-29. doi: 10.1007/s11154-011-9195-z.
Xue, Y., Karaplis, A. C., Hendry, G. N., et al. (2006). Exogenous 1,25-dihydroxyvitamin D3 exerts a skeletal anabolic effect and improves mineral ion homeostasis in mice that are homozygous for both the 1a-hydroxylase and parathyroid hormone null alleles. Endocrinology, 147, 4801-4810.
Zhong Y, Armbrecht HJ, Christakos S. Calcitonin, a regulator of the 25-hydroxyvitamin D3 1alphahydroxylase gene. J Biol Chem 2009;284:11059-69. [PubMed: 19261615]
Zordoky B. N. M., El-Kadi A. O. S. Role of NF-κB in the regulation of cytochrome P450 enzymes //Current drug metabolism. - 2009. - T. 10. - 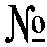 . 2. - C. 164-178

## Claims

1. *Passiflora Edulis* extract for use in the stimulation and/or induction of vitamin D3 synthesis from vitamin D3 precursors in a subject, wherein said subject is preferably a human.

2. The extract for use of claim 1, wherein said stimulation and/or induction of vitamin D3 synthesis is in the presence and/or absence of ultraviolet B rays
- in the skin of said subject, preferably in the skin keratinocytes, and/or
- in the oral cavity of said subject, preferably in the fibroblasts of the oral soft tissue, including gingival fibroblasts in the oral cavity of said subject.

3. *Passiflora Edulis* extract for use of any of the preceding claims, wherein said subject is suffering from or is at risk of suffering from vitamin D hypovitamimosis and/or symptoms associated with said vitamin D hypovitamimosis selected from the group consisting of atopic or seborrheic dermatitis, xerosis, eczema, psoriasis, acne, dry skin, cracks and skin ruptures, hyperpigmentation, erythema, vitiligo, alopecia, skin carcinogenesis, oral cancer, gingivitis, rachitic teeth, periodontitis and gum bleeding.

4. *Passiflora Edulis* extract for use in the treatment or prevention of vitamin D hypovitamimosis and/or symptoms associated with said vitamin D hypovitamimosis selected from the group consisting of atopic or seborrheic dermatitis, xerosis, eczema, psoriasis, acne, dry skin, cracks and skin ruptures, hyperpigmentation, erythema, vitiligo, alopecia, skin carcinogenesis, oral cancer, gingivitis, rachitic teeth, periodontitis and gum bleeding in a subject, wherein said subject is preferably a human.

5. The extract for use of any of the preceding claims, wherein said extract is specified by at least one of the following numbers according to the European commission database for information on cosmetic substances and ingredients (CosIng) database:
- CAS # 91770-48-8,
- EC # 294-833-9.

6. The extract for use of any of the preceding claims, wherein said extract is for application in therapeutical concentration of 10.01 mg/mL or less, preferably in a range of 1.00 mg/mL to 10.01 mg/mL.

7. The extract for use of any of the preceding claims, wherein said extract is comprised by a cosmetic and/or medical care product, preferably i) a skin care product for topical application on said subject's skin, or ii) an oral and/or dental care product formulated for topical application in the oral cavity of said subject.

8. The extract for use of any of the preceding claims, wherein said extract or cosmetic and/or medical care product comprising said extract is a formulation selected from the following: mouth wash, film, aerosol, suspension, solution, tincture, mask, serum, cream, paste, including toothpaste, lotion, ointment, gel, balsam, foam.

9. The extract for use of any of the preceding claims, wherein said extract produced by a method comprising the following steps:
a) providing whole *Passiflora Edulis* fruits and drying and grounding said dried fruits into a powder;
b) providing a glycerine-water extraction solvent comprising 30-50% glycerine by volume;
c) combining said powder with said extraction solvent and reflux extraction for 2-4 h at 60-80°C and at a 1:10-1:20 w/v solid-to-solvent ratio, followed by
d) filtration and partial water evaporation under reduced pressure at 40-60°C for extract concentration.

10. Skin or oral and/or dental care product formulated for topical application on the skin and/or in the oral cavity of a subject, preferably a human subject, said product comprising *Passiflora Edulis* extract.

11. The product of claim 10, wherein said extract is specified by at least one of the following numbers according to the European commission database for information on cosmetic substances and ingredients (CosIng) database:
- CAS # 91770-48-8,
- EC # 294-833-9.

12. The product of claim 10 or claim 11, wherein said product comprises said extract in a concentration of 10.01 mg/mL or less, preferably in a range of 1.00 mg/mL to 10.01 mg/mL.

13. The product of any of claims 10 to 12, wherein said product is a formulation selected from the following: mouth wash, film, aerosol, suspension, solution, tincture, mask, serum, cream, paste, including toothpaste, lotion, ointment, gel, balsam, foam.

14. The product of any of claims 10 to 13, wherein said extract in said product has been produced by a method comprising the following steps:
a) providing whole *Passiflora Edulis* fruits and drying and grounding said dried fruits into a powder;
b) providing a glycerine-water extraction solvent comprising 30-50% glycerine by volume;
c) combining said powder with said extraction solvent and reflux extraction for 2-4 h at 60-80°C and at a 1: 10-1:20 w/v solid-to-solvent ratio, followed by
d) filtration and partial water evaporation under reduced pressure at 40-60°C for extract concentration.

15. A method of topical treatment of the skin and/or oral cavity of a living subject wherein said method comprises a topical application of said skin and/or oral cavity with a product of any of claims 10-14.
